# FASCICULE DE BREVET EUROPEEN

(11) **EP 1 865 851 B1**
(45) Date de publication et mention de la délivrance du brevet: **04.08.2010**
(21) Numéro de dépôt: 06743598.2
(22) Date de dépôt: 24.03.2006
(51) Int. Cl.: A61B 6/00, A61N 5/10, A61N 5/00

(54) **DISPOSITIF D'AUTOCONTRÔLÉ DE SA RESPIRATION PAR UN INDIVIDU EN VUE DE L'ASSISTANCE AU PILOTAGE D'UNE UNITÉ DE RADIOTHÉRAPIE OU D'IMAGERIE**
VORRICHTUNG ZUR SELBSTKONTROLLIERTEN ATMUNG EINER PERSON UND ZUR UNTERSTÜTZUNG DER ÜBERWACHUNG EINER STRAHLENTHERAPIE- ODER ABBILDUNGSEINHEIT
DEVICE FOR SELF-CONTROLLED BREATHING BY A PERSON FOR ASSISTING MONITORING OF A RADIATION THERAPY OR IMAGING UNIT

(30) Priorité: 04.04.2005 FR 0503282; 12.04.2005 US 670282 P
(43) Date de publication de la demande: 19.12.2007
(73) Titulaire: Le Corre, Patrick, F-31600 Muret (US)
(72) Inventeur: Le Corre, Patrick, F-31600 Muret (US)
(74) Mandataire: Cabinet BARRE LAFORGUE & associés
(86) Numéro de dépôt international: PCT/FR2006/000654
(87) Numéro de publication internationale: WO 2006/106202

(56) Documents cités:
- FR-A- 2 823 679
- US-A- 5 949 080
- US-B1- 6 597 939
- US-B1- 6 633 775

## Description

L'invention concerne un dispositif d'autocontrôle de sa respiration par un individu en vue d'assister le pilotage d'une unité de radiothérapie pour le traitement de tumeurs ou d'imagerie.

Il s'agit de tumeurs situées au niveau de la cage thoracique (poumons, seins, etc.) ou de tumeurs dont la position varie en fonction de l'expansion thoracique (foie, etc.).

L'une des difficultés majeures de la radiothérapie est d'irradier en rayonnements, notamment en rayonnements X, la tumeur en limitant au maximum l'irradiation de tissus sains situés au voisinage immédiat de la tumeur. Aussi, dans le cas du traitement d'une tumeur, le patient est due manière classique invité à gonfler au maximum ses poumons afin d'atteindre un état dans lequel la quantité de tissu par unité de surface est minimal et la pénétration des rayonnements dans les tissus infectés à traiter la plus uniforme possible. Lorsque le gonflement est jugé satisfaisant par le praticien, le patient est invité à bloquer sa respiration et à rentrer en apnée le temps de l'irradiation.

La capacité de ventilation pulmonaire qui se traduit par la capacité à gonfler ou à contracter les poumons varie d'un individu à l'autre. De plus, tous les individus ne sont pas égaux devant le stress ce qui engendre chez certains individus des difficultés à maîtriser la respiration et donc à stabiliser l'organe à traiter.

Aussi, différents dispositifs ont été développés pour permettre, d'une part d'assister le praticien pour le pilotage d'une unité de radiothérapie et, d'autre part d'aider le patient à atteindre des niveaux de gonflement ou de contraction des poumons suffisants pour le traitement qu'il doit subir.

Par exemple, FR-2.823.679 décrit un dispositif de pilotage d'une unité de radiothérapie comprenant des moyens de mesure de l'expansion thoracique d'un patient et une unité de commande adaptée pour, dans un premier temps, comparer les valeurs de l'expansion thoracique mesurées avec des valeurs mémorisées de l'expansion thoracique au repos du patient, et dans un second temps, déclencher l'irradiation dès que les valeurs mesurées atteignent des valeurs limites d'expansion thoracique préalablement établies et correspondant à un gonflement des poumons adapté à une thérapie de qualité. Ce dispositif est par ailleurs doté de moyens de visualisation permettant au patient et au praticien de visualiser l'évolution dans le temps de l'expansion thoracique ainsi que les valeurs de déclenchement à atteindre pour pouvoir subir la radiothérapie.

Si les qualités de ce dispositif sont prouvées, notamment par une utilisation largement répandue dans les centres hospitaliers, il ne permet pas en revanche d'aider les patients les plus stressés à atteindre les niveaux de déclenchement recherchés. En effet, un patient stressé qui voit sur un dispositif de visualisation que son niveau d'expansion pulmonaire est éloigné de la valeur limite à atteindre a par expérience tendance à forcer sa respiration, ce qui dans le meilleur des cas le conduit à atteindre ce niveau d'expansion pulmonaire de déclenchement, mais sans pouvoir tenir une apnée suffisamment longue et stable, condition nécessaire pour une irradiation sécurisée.

Le dispositif décrit dans WO 99/43260 est un dispositif d'assistance au déclenchement de l'irradiation par l'analyse d'un certain nombre de paramètres reflétant l'état de la respiration d'un patient - débit d'air, pression, volume estimé des poumons, concentration en CO₂- qui alimentent l'entrée d'une unité de commande qui est adaptée pour déclencher, sous contrôle du praticien, l'irradiation, lorsque la compilation de ces paramètres révèle des conditions nominales d'expansion pulmonaire pour le patient considéré.

Ce dispositif permet d'assister le praticien dans son choix du moment propice pour déclencher l'irradiation, mais ne permet pas au patient de visualiser, ni de contrôler l'instant d'irradiation.

L'invention vise à pallier les inconvénients précités et à améliorer la sérénité d'un individu amené à suivre des séances de radiothérapies en proposant un dispositif d'autocontrôle de sa respiration.

Un autre objectif de l'invention est de fournir un dispositif d'autocontrôle de sa respiration par un individu qui permette de suivre et de contrôler l'évolution dans le temps des cycles de respiration.

Un autre objectif de l'invention est de fournir un dispositif permettant de coopérer à l'obtention de meilleures observations par une unité d'imagerie en améliorant et en contrôlant les apnées réalisées par un individu.

Un autre objectif de l'invention est de fournir un dispositif réactif aux variations des cycles de respiration d'un individu de manière à ce que ce dernier parvienne à atteindre progressivement un état prédéterminé.

Pour ce faire, l'invention concerne un dispositif d'autocontrôle de sa respiration par un individu en vue de l'assistance au pilotage d'une unité de radiothérapie ou d'imagerie comprenant :
- des moyens d'acquisition d'au moins un signal, dit signal de volume courant métabolique, représentatif de la variation du volume d'air contenu dans les poumons d'un individu au cours de ses cycles de respiration,
- une unité de traitement des signaux reliée auxdits moyens d'acquisition, ladite unité de traitement comprenant des moyens d'accès à au moins une valeur, dite valeur de repos, représentative pour un individu d'un cycle de respiration au repos et d'au moins une valeur, dite valeur de consigne, représentative pour un individu d'une inspiration et/ou d'une expiration cible prédéterminée,
- au moins une interface de communication avec un individu reliée à ladite unité de traitement, ladite interface de communication comprenant des moyens de communication aptes à communiquer un signal à l'individu.

Le dispositif d'autocontrôle selon l'invention est caractérisé en ce que l'unité de traitement des signaux :
- comprend des moyens de détection d'une respiration régulière au repos par comparaison dudit signal de volume courant métabolique à ladite (aux dites) valeur(s) de repos,
- et est adaptée pour commander lesdits moyens de communication afin que ces derniers émettent un signal représentatif de ladite (desdites) valeur(s) de consigne si ladite unité de traitement des signaux détecte une respiration régulière au repos.

Un dispositif conforme à l'invention permet d'une part de détecter une respiration régulière au repos propice à initier une inspiration ou une expiration prédéterminée et d'autre part de communiquer à un individu le niveau de l'inspiration ou de l'expiration cible prédéterminée. Aussi, un dispositif conforme à l'invention permet de décomposer en deux temps la mise en condition d'un individu préalablement à une séance de radiothérapie : un premier temps correspondant à une phase de respiration libre, au propre rythme de l'individu, qui permet à ce dernier de retrouver son calme et sa sérénité est un deuxième temps qui guide l'individu vers une inspiration et/ou une expiration cible prédéterminée(s). Cet accompagnement s'effectue par la communication d'un signal qui, d'une part, informe l'individu que les conditions nominales de respiration sont atteintes, et d'autre part, matérialise les niveaux de respiration à atteindre compatibles avec une radiothérapie. Cette mise en condition en deux temps permet de relaxer l'individu par une autogestion de sa respiration. De patient, il devient acteur et maître de sa préparation à la séance de radiothérapie ultérieure.

Par ailleurs, un dispositif conforme à l'invention permet également, lorsqu'il est associé à une unité d'imagerie, de participer à l'obtention de clichés de qualité par ladite unité d'imagerie, de par la qualité des inspirations et/ou expirations réalisées par un individu utilisateur du dispositif.

Avantageusement et selon l'invention, l'unité de traitement des signaux :
- comprend des moyens de détection d'une respiration non régulière et des moyens de détection d'une inspiration et/ou d'une expiration atteignant la (les) valeur(s) de consigne, par comparaison dudit signal de volume courant métabolique respectivement à ladite (auxdites) valeur(s) de repos et à ladite (auxdites) valeur(s) de consigne,
- et est adapté pour commander lesdits moyens de communication afin que ces derniers n'émettent aucun signal représentatif de ladite (desdites) valeur(s) de consigne si ladite unité de traitement détecte une respiration non régulière et si aucune inspiration et/ou expiration atteignant la (les) valeur(s) de consigne n'est (ne sont) détectée(s).

Ainsi, un dispositif conforme à l'invention est doté de moyens adaptés pour qu'une respiration régulière au repos engendre l'émission d'un signal représentatif d'au moins une valeur de consigne et qu'une respiration qui se dérègle et devienne non régulière suspende l'émission dudit signal interdisant toute tentative d'inspiration et/ou d'expiration cible et favorisant un retour au calme rapide.

Avantageusement et selon l'invention, la respiration régulière au repos est définie par au moins trois cycles de respiration au repos consécutifs et la respiration non régulière est définie par moins de trois cycles de respiration au repos consécutifs.

Par expérience, les trois cycles de respiration au repos permettent de garantir que l'individu est en phase de respiration régulière au repos. En variante, la respiration régulière au repos est définie par au moins cinq cycles de respiration régulière au repos.

Avantageusement et selon l'invention, l'unité de traitement est adaptée pour commander les moyens de communication afin que ces derniers émettent en continu un signal corrélé au signal de volume courant métabolique.

En variante, le signal est également communiqué à un opérateur extérieur afin qu'il puisse suivre l'évolution du signal dans le temps.

Avantageusement et selon l'invention, les moyens d'acquisition dudit signal de volume courant métabolique comprennent un spiromètre.

Le spiromètre est en effet le meilleur moyen de mesure de la variation du volume d'air contenu dans les poumons d'un individu au cours de ses cycles de respiration. De plus, un spiromètre permet d'établir une corrélation entre une expansion thoracique, c'est-à-dire une expiration et/ou une inspiration donnée et le volume d'air correspondant. D'autre part, un spiromètre permet avantageusement d'établir une corrélation précise entre le volume d'air contenu dans les poumons d'un individu et la position de l'organe à traiter. Il est donc possible avec ce type d'instrument de reproduire sensiblement à l'identique le positionnement de l'organe à traiter d'une séance à l'autre.

Avantageusement et selon l'invention, les valeurs de repos et de consigne représentatives d'un cycle de respiration au repos et d'une inspiration et/ou d'une expiration cible prédéterminée(s) sont respectivement le niveau ventilatoire de repos en fin de cycle de respiration au repos et le volume d'air contenu dans les poumons d'un individu correspondant à l'inspiration et/ou l'expiration cible prédéterminée(s).

Avantageusement et selon l'invention, les moyens de communication comprennent des moyens d'affichage adaptés pour afficher, en tant que signal corrélé au signal de volume courant métabolique, une courbe représentative de la variation du volume d'air contenu dans les poumons d'un individu au cours de sa respiration, et pour afficher en tant que signal représentatif de la (des) valeur(s) de consigne, une (des) barre(s) positionnée(s) à la (aux) valeur(s) de consigne en superposition de la courbe, de manière à ce qu'un individu puisse visualiser l'inspiration et/ou l'expiration cible prédéterminée(s).

Un signal visuel affiché sous forme d'une courbe sur laquelle vient s'ajouter une barre positionnée à la valeur de consigne est une information simple à interpréter par un individu et efficace pour transmettre à ce dernier l'état de sa respiration et la valeur de consigne.

En variante ou en combinaison, les moyens de communication comprennent des moyens de communications sonores adaptés pour émettre en tant que signal corrélé au signal de volume courant métabolique, un signal dont la fréquence est corrélée au signal de volume courant métabolique, et émettre en tant que signal représentatif de la (des) valeur(s) de consigne, un autre signal de fréquence prédéterminée.

Cette variante constitue un moyen efficace d'adapter un dispositif conforme à l'invention à l'autocontrôle de sa respiration par un individu malvoyant.

Avantageusement et selon l'invention, les moyens d'affichage comprennent des lunettes dotées d'au moins un écran à cristaux liquides et adaptées pour être portées par l'individu.

Ces lunettes permettent à un individu de suivre l'évolution de la variation du signal de volume courant métabolique ainsi que les niveaux d'inspiration et/ou d'expiration cibles à atteindre lorsque ces derniers sont affichés.

En variante ou en combinaison, les moyens d'affichage comprennent des écrans agencés au voisinage immédiat de l'individu de manière à ce que ce dernier puisse visualiser les signaux émis par les moyens de communications.

Avantageusement et selon l'invention, les moyens d'accès aux valeurs de repos et de consigne comprennent des moyens de mémorisation.

Avantageusement et selon l'invention, ces moyens de mémorisation comprennent au moins un dispositif de mémoire externe.

Avantageusement et selon l'invention, ces moyens de mémorisation comprennent en outre des plages de valeurs de repos et de consigne représentative respectivement d'une marge de mesure par rapport aux mesures de l'expansion thoracique au repos et d'une marge de mesure par rapport aux mesures de l'inspiration et/ou l'expiration cible.

Les plages de mesure sont avantageusement calculées de telle manière que la tolérance par rapport à la valeur nominale de repos et la valeur nominale d'inspiration ou d'expiration est de plus ou moins 0,1 litre. Elle a pour objectif de prendre en compte les variations possibles d'un cycle de respiration à l'autre.

Ces plages de valeurs de repos et de consigne sont paramétrables.

Un dispositif conforme à l'invention est avantageusement relié à une unité de commande adaptée pour commander une irradiation d'un individu par une unité de radiothérapie si les moyens de communication de l'interface de communication ont communiqué ledit signal représentatif de la (des) valeur(s) de consigne et si les mesures d'expansion thoracique de l'individu correspondent à la (aux) valeur(s) de consigne prédéterminée(s).

Un dispositif conforme à l'invention est également avantageusement utilisé avec une unité d'imagerie de manière à déterminer les valeurs de consigne compatibles avec une irradiation de qualité pour la tumeur concernée. En pratique, le dispositif conforme à l'invention sera couplé, dans une première phase d'apprentissage préalable, à une unité d'imagerie, et dans une seconde phase, sera relié à une unité de commande adaptée pour commander une unité de radiothérapie.

L'invention s'étend à un procédé mis en oeuvre par et dans un dispositif selon l'invention. L'invention s'étend ainsi à un procédé d'autocontrôle de sa respiration par un individu en vue de l'assistance au pilotage d'une unité de radiothérapie ou d'imagerie comprenant les étapes consistant à :
- faire l'acquisition d'au moins un signal, dit signal de volume courant métabolique, représentatif de la variation du volume d'air contenu dans les poumons d'un individu au cours de ses cycles de respiration,
- accéder à au moins une valeur dite de repos représentative pour un individu d'un cycle de respiration au repos et d'au moins une valeur dite de consigne représentative pour un individu d'une inspiration et/ou d'une expiration cible prédéterminée.

Le procédé selon l'invention est caractérisé en ce qu'il comprend les étapes consistant à :
- détecter une respiration régulière au repos par comparaison du volume courant métabolique à ladite (aux dites) valeur(s) de repos,
- et communiquer à l'individu un signal représentatif de ladite (desdites) valeur(s) de consigne si une respiration régulière au repos est détectée.

Avantageusement et selon l'invention, le procédé comprend en outre les étapes consistant à :
- détecter une respiration non régulière par comparaison du signal de volume courant métabolique à ladite (auxdites) valeur(s) de repos,
- détecter une inspiration et/ou une expiration cible prédéterminée(s) par comparaison du signal de volume courant métabolique à ladite (auxdites) valeur(s) de consigne,
- et cesser de communiquer ledit signal représentatif de ladite (desdites) valeur(s) de consigne si une respiration non régulière est détectée et si aucune inspiration et/ou expiration cible n'a (n'ont) été détectée(s).

Avantageusement et selon l'invention, la respiration régulière au repos est définie par au moins trois cycles de respiration au repos consécutifs et la respiration non régulière est définie par moins de trois cycles de respiration au repos consécutifs.

Avantageusement et selon l'invention, le procédé comprend l'étape consistant à communiquer en continu à l'individu un signal corrélé audit signal de volume courant métabolique.

Avantageusement et selon l'invention, l'étape d'acquisition du signal de volume courant métabolique est effectuée à l'aide d'un spiromètre

Avantageusement et selon l'invention, l'étape consistant à communiquer à l'individu un signal corrélé au signal de volume courant métabolique consiste à afficher une courbe représentative de la variation du volume d'air contenu dans les poumons d'un individu au cours de sa respiration.

Avantageusement et selon l'invention, l'étape consistant à communiquer un signal représentatif de ladite (desdites) valeur(s) de consigne consiste à afficher, en superposition de la courbe représentative de la variation de volume d'air contenu dans les poumons d'un individu au cours de sa respiration, une(des) barre(s) positionnée(s) à ladite (auxdites) valeur(s) de consigne de manière à ce qu'un individu puisse visualiser ladite inspiration et/ou ladite expiration cible prédéterminée(s).

Avantageusement et selon l'invention, l'étape consistant à communiquer à l'individu un signal corrélé au signal de volume courant métabolique s'effectue à l'aide de lunettes dotées d'au moins un écran à cristaux liquides et adaptées pour être portées par un individu.

En variante et selon l'invention, l'étape consistant à communiquer à l'individu un signal corrélé au signal de volume courant métabolique comprend en outre une étape consistant à émettre un signal sonore dont la fréquence est corrélée audit signal de volume courant métabolique.

Avantageusement et selon cette variante, l'étape consistant à communiquer un signal représentatif de ladite (desdites) valeur(s) de consigne consiste à émettre un signal de fréquence prédéterminée.

Avantageusement et selon l'invention, l'étape consistant à accéder auxdites valeurs de repos et de consigne consiste à accéder à des moyens de mémorisation.

L'invention concerne également un dispositif apte à mettre en oeuvre un procédé selon l'invention.

L'invention concerne en outre un dispositif d'autocontrôle de sa respiration par un individu caractérisé en combinaison par tout ou partie des caractéristiques mentionnées ci-dessus ou ci-après.

D'autres caractéristiques, buts et avantages de l'invention apparaîtront à la lecture de la description suivante qui présente à titre d'exemple non limitatif un mode de réalisation de l'invention, en référence aux dessins annexés ; sur ces dessins :
- la figure 1 est un schéma synoptique d'un dispositif de contrôle de la respiration d'un individu en vue de l'assistance au pilotage d'une unité de radiothérapie,
- la figure 2 est un graphique représentant une courbe respiratoire d'un individu telle que présentée à ce dernier selon un mode de réalisation de la présente invention.

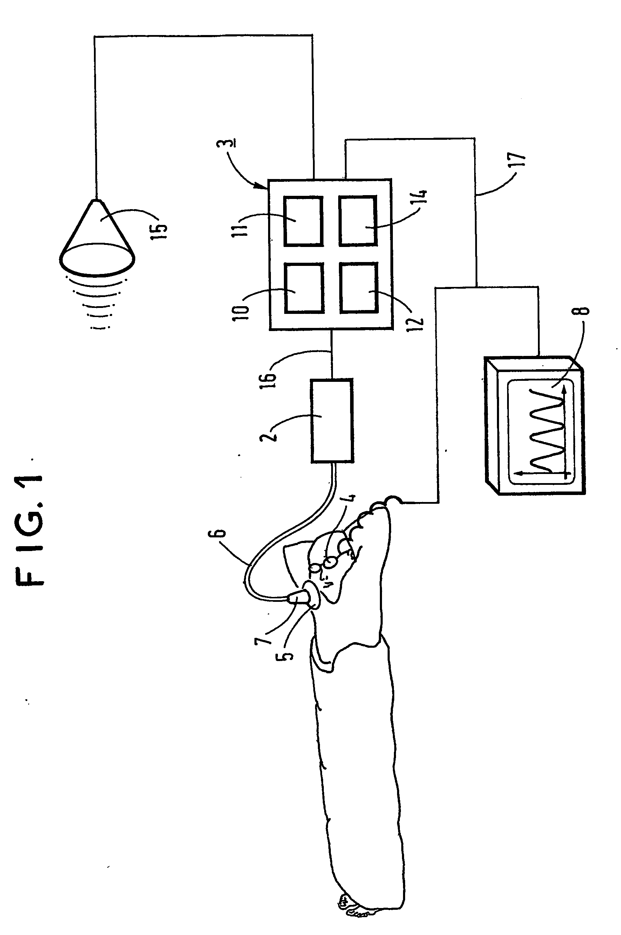

Le dispositif d'assistance au pilotage représenté sur la figure 1 comprend un spiromètre 2 du type pneumotachographe, comportant un capteur intégrateur des différences de pression du flux respiratoire, et adapté pour délivrer un signal électrique représentatif du volume d'air correspondant à l'expansion thoracique.

Selon le mode de réalisation de la figure 1, le spiromètre 2 est relié à un embout 5 buccal par un tuyau 6 souple au bout duquel est inséré un filtre 7 bactérien interchangeable. Le spiromètre 2 incorpore également des moyens de chauffage du capteur intégrateur à une température stabilisée, telle qu'une résistance électrique, alimentés en permanence de manière à éliminer l'humidité produite par la respiration de l'individu susceptible d'entraîner le cas contraire des erreurs importantes dans le calcul des volumes.

Le spiromètre 2 utilisé est selon le mode de réalisation de la figure 1 un spiromètre tel que décrit dans FR-2.823.679.

Le spiromètre 2 est relié, selon un mode de réalisation de la présente invention, à une unité de traitement 3 des signaux par l'intermédiaire d'une liaison 16 du type USB. L'unité de traitement 3 est selon le mode de réalisation de la figure 1 constituée d'une unité centrale de micro-ordinateur mais peut selon d'autres modes de réalisation comprendre un DSP ou tout autre système du type calculateur et/ou processeur adapté(s) pour traiter des données numériques ou analogiques.

L'unité de traitement 3 comprend en outre des moyens d'accès à des valeurs respectivement de repos et de consigne. On entend de manière classique par moyens d'accès à des valeurs, des moyens et des voies d'obtention de ces valeurs. L'accès à une valeur est donc la recherche et le moyen d'obtention de cette valeur à partir d'une mémoire, ou dans tout élément de stockage, qui couvre aussi bien les disques, les bandes ou tout support externe, que les mémoires caches et les mémoires vives des unités centrales.

Selon le mode de réalisation de la figure 1, les moyens d'accès sont constitués de moyens 14 de mémorisation qui, de préférence, sont des mémoires de masse tel qu'un disque dur de micro-ordinateur. Selon d'autres modes de réalisation, ces moyens 14 de mémorisation comprennent au moins un support d'enregistrement amovible, du type disquette, disque magnéto-optique, disque optique, clé à mémoire électronique du type USB notamment, carte à mémoire électronique, etc.

L'unité de traitement 3 comprend également des moyens 10 de détection d'une respiration régulière au repos. Ces moyens de détection sont, selon un mode de réalisation de la présente invention, des moyens numériques adaptés pour échantillonner le signal acquis par le spiromètre, comparer chaque échantillon à la valeur de repos, mémoriser le résultat de la comparaison et émettre un signal de détection d'une respiration régulière au repos si les résultats mémorisés de la comparaison présentent au moins trois concordances successives.

Selon un autre mode de réalisation, ces moyens 10 de détection peuvent être des moyens de détection analogiques ou des moyens combinés analogiques et numériques.

L'unité de traitement 3 est reliée à une interface de communication qui selon le mode de réalisation de la figure 1 comprend un dispositif d'affichage qui est constitué d'un écran 8 de micro-ordinateur et d'une paire de lunettes 4 comportant deux écrans à cristaux liquides. Cette interface graphique de communication permet, sur commande de l'unité de traitement 3 des signaux, d'une part d'afficher une courbe 13 représentative des cycles de respiration d'un individu, et, d'autre part d'afficher en sus, si les moyens de détection ont détecté une respiration régulière au repos, une barre 9 positionnée à chacune des valeurs de consigne. Selon un autre mode de réalisation, les lunettes à cristaux liquides sont remplacées par des petits écrans agencés au voisinage de l'individu.

L'échange d'informations entre l'unité de traitement 3 des signaux et le dispositif d'affichage s'effectue par l'intermédiaire de liaisons filaires séries 17, par exemple du type USB.

L'utilisation d'un dispositif de contrôle de la respiration d'un individu selon l'invention se déroule tel que décrit ci-dessous.

L'étape de préparation consiste essentiellement à déterminer et mémoriser selon un mode de réalisation de l'invention, au moins une valeur dite de repos, représentative d'un cycle de respiration au repos et au moins une valeur dite de consigne, représentative d'un niveau d'inspiration et/ou d'expiration cible. Ce niveau cible est déterminé par un praticien et correspond à un niveau permettant de procéder ultérieurement à une radiothérapie de qualité.

Afin de procéder à une radiothérapie, d'autres étapes supplémentaires sont nécessaires, mais sortent du cadre de cette invention qui concerne uniquement le dispositif d'autocontrôle de sa respiration par un individu.

La première valeur est déterminée lorsque l'individu respire normalement au repos par la mesure de son volume courant métabolique et la mémorisation d'une valeur de repos Vr représentative du niveau ventilatoire de repos de l'individu en fin de cycle au repos.

La deuxième valeur est quant à elle déterminée lorsque l'individu effectue une inspiration ou une expiration profonde et en relevant le niveau d'inspiration et/ou d'expiration pour lequel la tumeur se trouve dans la meilleure position en vue d'un traitement en radiothérapie ultérieur. La valeur mémorisée dite de consigne Va correspond donc au niveau d'inspiration et/ou d'expiration pour lequel sera ensuite déclenchée l'irradiation lorsque l'individu effectuera une apnée à ce niveau en vue d'un traitement en radiothérapie ultérieur. Pour aider à la détermination de cette valeur, le dispositif conforme à l'invention est utilement couplé à une unité d'imagerie du type scanner afin que le praticien puisse visualiser la position de la tumeur et déterminer quelle inspiration ou expiration conduit au meilleur positionnement de l'organe à traiter ultérieurement.

Selon un mode de réalisation, les valeurs mesurées le sont avec une marge de 0,1 litre. Ainsi, les plages de valeurs [Vr-0,1ℓ, Vr+0,1ℓ] est [Va-0,1ℓ, Va+0,1ℓ] sont dites respectivement zone 18 de stabilité et zone 19 de consigne. Les marges de mesures sont paramétrables et peuvent être adaptées à l'individu utilisateur du dispositif. Ces marges de mesure peuvent ne pas être symétriques par rapport à la valeur nominale.

Ces valeurs sont stockées selon le mode de réalisation de la figure 1 dans une mémoire 14 de micro-ordinateur ou, selon d'autres modes de réalisation de la présente invention, stockées sur des supports amovibles.

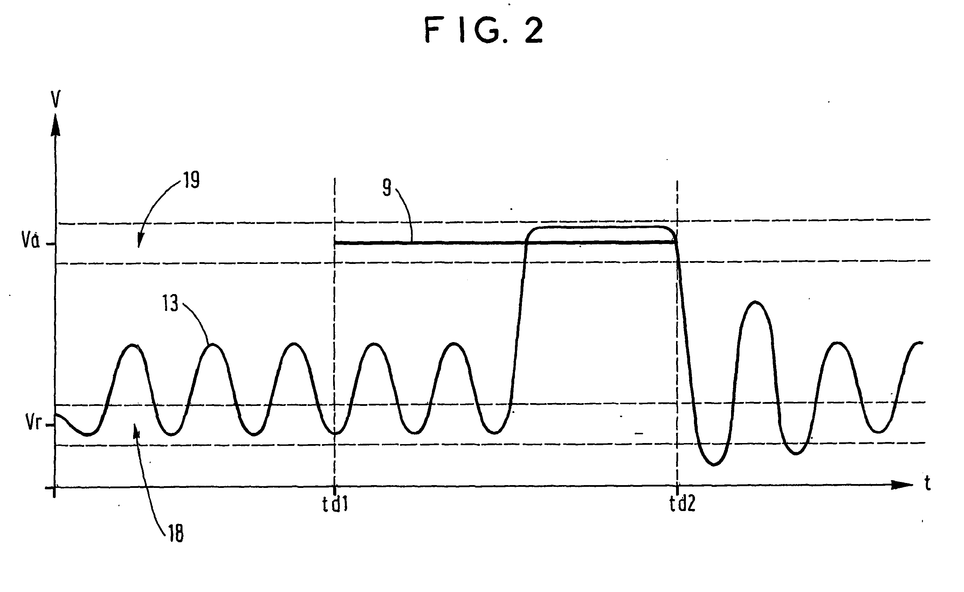

La figure 2 présente une courbe 13 représentative du volume d'air dans les poumons d'un individu au cours de ses cycles de respiration. L'axe des ordonnées représente le volume et l'axe des abscisses représente le temps. Vr représente le niveau ventilatoire au repos qui sert de référence pour détecter un cycle de respiration au repos et Va représente le volume d'air correspondant à une inspiration cible. Les zones de stabilité 18 et de consigne 19 sont représentées par des pointillés. La référence td1 représente l'instant à partir duquel l'unité de traitement 3 des signaux détecte que l'individu a atteint une respiration régulière au repos définie par au moins trois cycles de respiration au repos. A partir de cet instant, vient se superposer à la courbe, une barre 9 positionnée à la valeur de consigne, autrement dit au volume Va. La référence td2 représente l'instant à partir duquel l'unité de traitement 3 des signaux a détecté la fin de l'apnée et une respiration non régulière au repos, ce qui provoque la disparition de la barre 9 positionnée à la valeur Va.

L'affichage et la disparition de la barre 9 dépendent des détections effectuées par les moyens de détection de l'unité de traitement 3 des signaux. Selon un mode de réalisation de la présente invention, ces moyens de détection 10, 11, 12 s'appuient sur l'architecture suivante : dans un premier temps, les moyens 10, 11, 12 de détection isolent les cycles de respiration (par la détection de deux maximums locaux séparés d'un minimum local ou de deux minimums locaux séparés d'un maximum local) et déterminent, dans un second temps, pour chaque cycle respiratoire isolé, si le niveau bas du cycle est situé dans la zone 18 de stabilité. Si trois cycles consécutifs présentent un niveau ventilatoire au repos appartenant à la zone de stabilité, la respiration régulière est détectée et la barre à la valeur de consigne est affichée sur les moyens d'affichage 4, 8.

Si le cycle suivant présente un niveau ventilatoire au repos qui sort de la zone de stabilité, la respiration non régulière est détectée et la barre 9 d'affichage s'efface. Selon un mode de réalisation de la présente invention, il faut attendre de nouveau trois cycles consécutifs présentant chacun un niveau ventilatoire au repos compris dans la zone de stabilité pour que la barre 9 positionnée à ladite valeur de consigne s'affiche à nouveau. Selon un autre mode de réalisation, si trois cycles de respiration au repos ont été détectés et que le quatrième est hors de la zone de stabilité, si le cinquième est à nouveau dans la zone de stabilité, alors la barre 9 s'affiche à nouveau, la respiration étant considérée comme régulière au repos.

Lorsque la barre 9 est affichée, et en cas de non détection d'un nouveau cycle de respiration régulière au repos, deux cas de figure peuvent se produire par ordre décroissant de priorité. Dans le premier cas de figure, les moyens 12 de détection détectent que le signal représentatif de la variation du volume d'air contenu dans les poumons de l'individu atteint la zone 19 de consigne ; dans ce cas, la barre 9 reste affichée jusqu'à ce que le signal quitte la zone de consigne. Dans le deuxième cas de figure, les moyens 11 de détection détectent une respiration non régulière ; dans ce cas, la barre 9 s'efface à moins que le premier cas de figure se produise également, auquel cas, comme mentionné ci-dessus, la barre reste affichée.

Le graphique de la figure 2 est selon le mode de réalisation de la figure 1 transmis à l'individu par l'intermédiaire des lunettes 4 à cristaux liquides ou d'écrans agencés au voisinage de l'individu, ce qui permet à ce dernier, utilisateur du dispositif conforme à l'invention, de contrôler sa respiration en temps réel et d'être le seul juge du moment où il procède à l'inspiration ou à l'expiration cible. En pratique, cette inspiration ou expiration cible est tenue dans le temps de manière à réaliser une apnée qui permette de maintenir la position de la tumeur dans des conditions compatibles avec une radiothérapie.

Selon un mode de réalisation de la présente invention, le dispositif d'autocontrôle est relié à une unité de commande adaptée pour déclencher sur ordre d'un praticien spécialisé l'irradiation de l'individu lorsque ce dernier a effectué une apnée dans les conditions précitées. Selon ce mode de réalisation, l'irradiation cesse dès que l'individu relâche son apnée.

Un dispositif d'autocontrôle conforme à l'invention permet ainsi à un individu de se placer rapidement dans des prédispositions psychologiques favorables par une gestion efficace de son stress, ce qui permet un déroulement efficace d'une séance de radiothérapie.

## Revendications

1. Dispositif d'autocontrôle de sa respiration par un individu en vue de l'assistance au pilotage d'une unité de radiothérapie ou d'imagerie comprenant :
- des moyens d'acquisition d'au moins un signal, dit signal de volume courant métabolique, représentatif de la variation du volume d'air contenu dans les poumons d'un individu au cours de ses cycles de respiration,
- une unité de traitement (3) des signaux reliée auxdits moyens d'acquisition, ladite unité de traitement (3) comprenant des moyens d'accès à au moins une valeur, dite valeur de repos, représentative pour un individu d'un cycle de respiration au repos et d'au moins une valeur, dite valeur de consigne, représentative pour un individu d'une inspiration et/ou d'une expiration cible prédéterminée,
- au moins une interface de communication avec un individu reliée à ladite unité de traitement (3), ladite interface de communication comprenant des moyens de communication aptes à communiquer un signal à l'individu,
ledit dispositif d'autocontrôle étant **caractérisé en ce que** l'unité de traitement (3) des signaux :
- comprend des moyens (10) de détection d'une respiration régulière au repos par comparaison du signal de volume courant métabolique à ladite/aux dites valeur/s de repos,
- et est adaptée pour commander lesdits moyens de communication afin que ces derniers émettent un signal représentatif de ladite /desdites valeur/s de consigne si ladite unité de traitement (3) des signaux détecte une respiration régulière au repos.

2. Dispositif d'autocontrôle selon la revendication 1, **caractérisé en ce que** l'unité de traitement (3) des signaux :
- comprend des moyens (11) de détection d'une respiration non régulière et des moyens (12) de détection d'une inspiration et/ou d'une expiration atteignant la/les valeur/s de consigne, par comparaison du signal de volume courant métabolique respectivement à ladite/auxdites valeur/s de repos et à ladite/auxdites valeur/s de consigne,
- et est adapté pour commander lesdits moyens de communication afin que ces derniers n'émettent aucun signal représentatif de ladite/desdites valeur/s de consigne si ladite unité de traitement (3) détecte une respiration non régulière et si aucune inspiration et/ou expiration atteignant la /les valeur/s de consigne n'est/ne sont détectée/s

3. Dispositif d'autocontrôle selon la revendication 2, **caractérisé en ce que** :
- ladite respiration régulière au repos est définie par au moins trois cycles de respiration au repos consécutifs,
- ladite respiration non régulière est définie par moins de trois cycles de respiration au repos.

4. Dispositif d'autocontrôle selon l'une des revendications précédentes, **caractérisé en ce que** l'unité de traitement (3) est adaptée pour commander lesdits moyens de communication afin que ces derniers émettent en continu un signal corrélé audit signal de volume courant métabolique.

5. Dispositif d'autocontrôle selon la revendication 4, **caractérisé en ce que** les moyens d'acquisition dudit signal de volume courant métabolique comprennent un spiromètre (2).

6. Dispositif d'autocontrôle selon la revendication 5, **caractérisé en ce que** lesdites valeurs de repos et de consigne représentatives d'un cycle de respiration au repos et d'une inspiration et/ou d'une expiration cible prédéterminée/s sont respectivement un niveau ventilatoire au repos d'un individu en fin de cycle de respiration au repos et un volume d'air contenu dans les poumons d'un individu correspondant à ladite inspiration et/ou ladite expiration cible prédéterminée/s.

7. Dispositif d'autocontrôle selon la revendication 6, **caractérisé en ce que** les moyens de communication comprennent des moyens (4, 8) d'affichage adaptés pour afficher, en tant que signal corrélé au signal de volume courant métabolique, une courbe (13) représentative de la variation du volume d'air contenu dans les poumons d'un individu au cours de sa respiration, et pour afficher en tant que signal représentatif de ladite/desdites, valeur/s de consigne , une/des barre/s ; (9) positionnée/s) à ladite/auxdites valeur/s de consigne en superposition de ladite courbe (13), de manière à ce qu'un individu puisse visualiser ladite inspiration et/ou ladite expiration cible prédéterminée/s.

8. Dispositif d'autocontrôle selon la revendication 7, **caractérisé en ce que** les moyens d'affichage comprennent des lunettes (4) dotées d'au moins un écran à cristaux liquides et adaptées pour être portées par l'individu.

9. Dispositif d'autocontrôle selon l'une des revendications précédentes, **caractérisé en ce que** les moyens de communication comprennent des moyens (15) de communications sonores adaptés pour émettre en tant que signal corrélé audit signal de volume courant métabolique, un signal dont la fréquence est corrélée audit signal de volume courant métabolique, et émettre en tant que signal représentatif de ladite/desdites: valeur/s de consigne, un autre signal de fréquence prédéterminée.

10. Dispositif d'autocontrôle selon l'une des revendications précédentes, **caractérisé en ce que** les moyens d'accès auxdites valeurs de repos et de consigne comprennent des moyens (14) de mémorisation.

11. Dispositif d'autocontrôle selon la revendication 10, **caractérisé en ce que** les moyens (14) de mémorisation comprennent au moins un dispositif de mémoire externe.

12. Dispositif d'autocontrôle selon l'une des revendications 10 ou 11, **caractérisé en ce que** les moyens (14) de mémorisation comprennent en outre une plage de valeurs de repos et de consigne représentative respectivement d'une marge de mesure par rapport aux mesures du niveau ventilatoire au repos et d'une marge de mesure par rapport aux mesures de l'inspiration et/ou l'expiration cible.

13. Ensemble formé d'un dispositif d'autocontrôle selon l'une des revendications précédentes et d'une unité de commande adaptée pour commander une irradiation d'un individu par une unité de radiothérapie si lesdits moyens de communication de ladite interface de communication dudit dispositif d'autocontrôle ont communiqué ledit signal représentatif de ladite/desdites valeur/s de consigne et si les mesures d'expansion thoracique de l'individu correspondent à ladite/auxdites valeur/s de consigne prédéterminée/s

## Claims

1. Device enabling an individual to self-monitor his breathing in order to assist in the control of a radiotherapy or imaging unit comprising
- means for acquiring at least one signal, known as the current metabolic volume signal, which is representative of the variation in the volume of air contained in the lungs of an individual during his breathing cycles,
- a signal-processing unit (3) connected to said acquisition means, said processing unit (3) comprising means for accessing at least one value, known as the rest value, which is representative for an individual of a breathing cycle during rest, and at least one value, known as the setpoint value, which is representative for an individual of a predetermined target inhalation and/or exhalation,
- at least one interface for communication with an individual, said communication interface being connected to said processing unit (3) and comprising communication means capable of communicating a signal to the individual,
said self-monitoring device being **characterised in that** the unit (3) for processing the signals
- comprises means (10) for detecting regular breathing during rest by comparing the current metabolic volume signal with said rest value(s)
- and is capable of controlling said communication means in order that said means transmit a signal that is representative of said setpoint value(s) if said signal-processing unit (3) detects regular breathing during rest.

2. Self-monitoring device according to claim 1, **characterised in that** the signal-processing unit (3):
- comprises means (11) for detecting non-regular breathing and means (12) for detecting inhalation and/or exhalation reaching the setpoint value(s), by comparing the current metabolic volume signal with said rest value(s) and with said setpoint values(s) respectively,
- and is capable of controlling said communication means in order that said means do not transmit a signal that is representative of said setpoint value(s) if said processing unit (3) detects non-regular breathing and if no inhalation and/or exhalation reaching the setpoint value(s) is/are detected.

3. Self-monitoring device according to claim 2, **characterised in that**:
- said regular breathing during rest is defined by at least three successive breathing cycles during rest,
- said non-regular breathing is defined by less than three breathing cycles during rest.

4. Self-monitoring device according to any one of the preceding claims, **characterised in that** the processing unit (3) is capable of controlling said communication means in order that said means continuously transmit a signal correlated with said current metabolic volume signal.

5. Self-monitoring device according to claim 4, **characterised in that** the means for acquiring said current metabolic volume signal comprise a spirometer (2).

6. Self-monitoring device according to claim 5, **characterised in that** said rest and setpoint values, which are representative of a breathing cycle during rest and of a predetermined target inhalation and/or exhalation, are a ventilation level when an individual is at rest at the end of his breathing cycle during rest and a volume of air contained in the lungs of an individual corresponding to said predetermined target inhalation and/or said predetermined target exhalation, respectively.

7. Self-monitoring device according to claim 6, **characterised in that** the communication means comprise display means (4, 8) capable of displaying, as a signal correlated with the current metabolic volume signal, a curve (13), which is representative of the variation in the volume of air contained in the lungs of an individual during his breathing, and of displaying, as a signal representing said setpoint value(s), one or more bars (9) positioned at said setpoint value(s) superimposed on said curve (13), thus enabling an individual to visualise said predetermined target inhalation and/or said predetermined target exhalation.

8. Self-monitoring device according to claim 7, **characterised in that** the display means comprise glasses (4) provided with at least one liquid crystal display and capable of being worn by the individual.

9. Self-monitoring device according to any one of the preceding claims, **characterised in that** the communication means comprise sound communication means (15) capable of transmitting, as a signal correlated with said current metabolic volume signal, a signal, the frequency of which is correlated with said current metabolic volume signal, and of transmitting, as a signal representing said setpoint value(s), another signal of predetermined frequency.

10. Self-monitoring device according to any one of the preceding claims, **characterised in that** the means for accessing said rest and setpoint values comprise storage means (14).

11. Self-monitoring device according to claim 10, **characterised in that** the storage means (14) comprise at least one external memory device.

12. Self-monitoring device according to either claim 10 or claim 11, **characterised in that** the storage means (14) also comprise a range of rest and setpoint values that is representative of a margin of measurement relative to the measurements of the ventilation level during rest and of a margin of measurement relative to the target inhalation and/or exhalation measurements, respectively.

13. Assembly consisting of a self-monitoring device according to any one of the preceding claims and of a control unit capable of controlling the irradiation of an individual by a radiotherapy unit if said communication means of said communication interface of said self-monitoring device have communicated said signal representing said setpoint value(s) and if the measurements of the expansion of the individual's thorax correspond to said predetermined setpoint value(s).

## Patentansprüche

1. Vorrichtung zur Selbstkontrolle seiner Atmung durch einen Patienten zwecks Unterstützung bei der Steuerung einer Röntgentherapie- oder Bildgebungseinheit, die Folgendes umfasst:
- Erfassungsmittel wenigstens eines Signals, bezeichnet als metabolisches aktuelles Volumensignal, das die Schwankung des Luftvolumens darstellt, das in den Lungen eines Patienten im Verlauf seiner Atmungszyklen enthalten ist,
- eine Behandlungseinheit (3) der mit den genannten Erfassungsmitteln verbundenen Signale, wobei die genannte Behandlungseinheit (3) Zugangsmittel zu wenigstens einem Wert umfasst, bezeichnet als Ruhewert, der bei einem Patienten einen Atmungszyklus im Ruhezustand darstellt, und wenigstens einem Wert, bezeichnet als Sollwert, der bei einem Patienten ein vorbestimmtes, gezielte Ein- und / oder Ausatmen darstellt,
- wenigstens eine Kommunikationsschnittstelle mit einem Patienten, die an die genannte Behandlungseinheit (3) angeschlossen ist, wobei die genannte Kommunikationsschnittstelle Kommunikationsmittel umfasst, die geeignet sind, dem Patienten ein Signal zu übermitteln,
wobei die genannte Vorrichtung zur Selbstkontrolle **dadurch gekennzeichnet ist, dass** die Behandlungseinheit (3) der Signale:
- Mittel (10) zur Erfassung einer regelmäßigen Atmung im Ruhezustand durch den Vergleich mit dem metabolischen aktuellen Volumensignal mit dem genannten (den genannten) Ruhewert(en) umfasst,
- und geeignet ist, die genannten Kommunikationsmittel zu steuern, damit diese ein Signal ausgeben, das den genannten Sollwert (die genannten Sollwerte) darstellt, wenn die genannte Behandlungseinheit (3) der Signale eine regelmäßige Atmung im Ruhezustand feststellt.

2. Vorrichtung zur Selbstkontrolle gemäß Anspruch 1, **dadurch gekennzeichnet, dass** die Behandlungseinheit (3) der Signale:
- Mittel (11) zur Erfassung einer nicht regelmäßigen Atmung und Mittel (12) zur Erfassung eines Ein- und / oder eines Ausatmens, das den Sollwert (die Sollwerte) erreicht, durch den Vergleich des metabolischen aktuellen Volumensignals jeweils mit dem genannten Ruhewert (den genannten Ruhewerten) und dem genannten Sollwert (den genannten Sollwerten) umfassst,
- und geeignet ist, um die genannten Kommunikationsmittel zu steuern, damit diese kein Signal ausgeben, das den genannten Sollwert (die genannten Sollwerte) darstellt, wenn die genannte Behandlungseinheit (3) eine nicht regelmäßige Atmung feststellt und wenn kein Ein- und / oder Ausatmen festgestellt wird / werden, das / die den Sollwert (die Sollwerte) erreicht / erreichen.

3. Vorrichtung zur Selbstkontrolle gemäß Anspruch 2, **dadurch gekennzeichnet, dass**:
- das genannte regelmäßige Einatmen im Ruhezustand durch wenigstens drei konsekutive Atmungszyklen im Ruhezustand definiert wird,
- die genannte nicht regelmäßige Atmung durch wenigstens drei Atmungszyklen im Ruhezustand definiert wird.

4. Vorrichtung zur Selbstkontrolle gemäß einem der vorherigen Ansprüche, **dadurch gekennzeichnet, dass** die Behandlungseinheit (3) geeignet ist, um die genannten Kommunikationsmittel zu steuern, damit diese kontinuierlich ein Signal ausgeben, das mit dem metabolischen, aktuellen Signal korreliert ist.

5. Vorrichtung zur Selbstkontrolle gemäß Anspruch 4, **dadurch gekennzeichnet, dass** die Erfassungsmittel des genannten metabolischen aktuellen Volumensignals ein Spirometer (2) umfassen.

6. Vorrichtung zur Selbstkontrolle gemäß Anspruch 5, **dadurch gekennzeichnet, dass** die genannten Ruhe- und Sollwerte, die einen Atmungszyklus im Ruhezustand und ein vorbestimmtes, gezieltes Ein- und / oder Ausatmen darstellen, jeweils ein Ventilationsniveau im Ruhezustand eines Patienten am Ende des Atmungszyklus im Ruhezustand und ein Luftvolumen sind, das in den Lungen eines Patienten enthalten ist und dem genannten vorbestimmten gezielten Ein- und / oder Ausatmen entspricht.

7. Vorrichtung zur Selbstkontrolle gemäß Anspruch 6, **dadurch gekennzeichnet, dass** die Kommunikationsmittel Anzeigemittel (4, 8) umfassen, die als mit dem metabolischen aktuellen Volumensignal korreliertes Signal zur Anzeige einer Kurve (13) geeignet sind, die die Schwankung des Luftvolumens darstellt, das in den Lungen eines Patienten im Verlauf seiner Atmung enthalten ist, und um als Signal, das den genannten Sollwert (die genannten Sollwerte) darstellt, zur Anzeige eines (mehrerer) Balken(s) (9), der (die) an dem genannten Sollwert (den genannten Sollwerten) in Überlagerung der genannten Kurve (13) derart positioniert ist (sind), dass ein Patient das genannte vorbestimmte, gezielte Ein- und / oder Ausatmen ansehen kann.

8. Vorrichtung zur Selbstkontrolle gemäß Anspruch 7, **dadurch gekennzeichnet, dass** die Anzeigemittel Brillen (4) umfassen, die mit wenigstens einem Flüssigkristallbildschirm ausgestattet und geeignet sind, um vom Patienten getragen zu werden.

9. Vorrichtung zur Selbstkontrolle gemäß einem der vorherigen Ansprüche, **dadurch gekennzeichnet, dass** die Kommunikationsmittel akustische Kommunikationsmittel (15) umfassen, die geeignet sind, um als mit dem metabolischen aktuellen Volumensignal korrelierten Signal ein Signal auszugeben, dessen Frequenz mit dem genannten metabolischen aktuellen Signal korreliert ist, und als Signal, das den genannten Sollwert (die genannten Sollwerte) darstellt, ein anderes vorbestimmte Frequenzsignal auszugeben.

10. Vorrichtung zur Selbstkontrolle gemäß einem der vorherigen Ansprüche, **dadurch gekennzeichnet, dass** die Zugangsmittel zu den genannten Ruhe- und Sollwerten Speichermittel (14) umfassen.

11. Vorrichtung zur Selbstkontrolle gemäß Anspruch 10, **dadurch gekennzeichnet, dass** die Speichermittel (14) wenigstens eine externe Speichervorrichtung umfassen.

12. Vorrichtung zur Selbstkontrolle gemäß Anspruch 10 oder 11, **dadurch gekennzeichnet, dass** die Speichermittel (14) darüber hinaus einen Ruhe- und Sollwertbereich umfassen, der jeweils einen Messbereich im Verhältnis zu den Messungen des Atmungsniveaus im Ruhezustand und einen Messbereich im Verhältnis zu den Messungen der gezielten Ein- und / oder Ausatmen darstellt.

13. Struktur, die aus einer Vorrichtung zur Selbstkontrolle gemäß einem der vorherigen Ansprüche und einer Steuereinheit gebildet wird, die geeignet ist, um eine Bestrahlung eines Patienten durch eine Röntgentherapieeinheit zu steuern, wenn die genannten Kommunikationsmittel der genannten Kommunikationsschnittstelle der genannten Vorrichtung zur Selbstkontrolle das genannte Signal übermittelt haben, das den genannten Sollwert (die genannten Sollwerte) darstellt und wenn die Messungen der Brustkastenerweiterung des Patienten dem genannten, vorbestimmten Sollwert (den genannten vorbestimmten Sollwerten) entsprechen.
